(19) Europäisches Patentamt / European Patent Office / Office européen des brevets

(11) **EP 3 616 749 A1**

(12) **EUROPEAN PATENT APPLICATION**
published in accordance with Art. 153(4) EPC

(43) Date of publication:
**04.03.2020 Bulletin 2020/10**

(51) Int Cl.:
**A61N 2/04** (2006.01)

(21) Application number: **17906792.1**

(22) Date of filing: **24.04.2017**

(86) International application number:
**PCT/JP2017/016169**

(87) International publication number:
**WO 2018/198160 (01.11.2018 Gazette 2018/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Cellpower Co., Ltd.**
**Kyoto-shi, Kyoto 600-8468 (JP)**

(72) Inventor: **SASAKI, Koji**
**Kyoto-shi**
**Kyoto 600-8468 (JP)**

(74) Representative: **Winter, Brandl, Fürniss, Hübner, Röss, Kaiser, Polte - Partnerschaft mbB**
**Patent- und Rechtsanwaltskanzlei**
**Alois-Steinecker-Strasse 22**
**85354 Freising (DE)**

(54) **MAGNETIC THERAPY APPARATUS**

(57) When a gate control device passes a current into a gate of a first thyristor while a DC power supply is in an on state, conduction of the first thyristor starts, and a current starts to flow through a coil ring. The flowing current is stored as a charge in a capacitor, and a flow of the current stops when a voltage generated by the charge stored in the capacitor becomes equal to a voltage applied by the DC power supply, so that a pulsed current flows through the coil ring once. A pulse width of the pulsed current is 0.5 msec or more and 5.0 msec or less. The pulsed current is passed through the coil ring at a relatively low frequency of 30 Hz or less. When the pulsed current flows through the coil ring, a pulsed magnetic field is generated. When the pulsed magnetic field is provided from the coil ring to body tissue of the human body, an electromotive force having a similar waveform to an action potential is generated in the body tissue.

FIG. 3

**Description**

Technical Field

The present invention relates to a magnetic therapy apparatus generating a pulsed magnetic field to induce a weak current in body tissue.

Background Art

Transcranical magnetic stimulation (TMS) is known as magnetic therapy to induce a weak current in body tissue through magnetic stimulation. For example, Patent Documents 1 and 2 propose a system to execute the TMS. The TMS is said to be effective mainly for neurological symptoms and psychiatric symptoms.

Prior Art Documents

Patent Documents

Patent Document 1: Japanese Patent Application Laid-Open No. 2015-77484
Patent Document 2: Japanese Unexamined Patent Application Publication (Translation of PCT Application) No. 2012-511387

Summary

Problem to be Solved by the Invention

However, a problem in that the TMS causes an action potential through magnetic stimulation to present severe discomfort has been pointed out.

The present invention has been conceived in view of the above-mentioned problem, and it is an object of the present invention to provide a magnetic therapy apparatus presenting less discomfort.

Means to Solve the Problem

To solve the above-mentioned problem, a first aspect of the present invention is a magnetic therapy apparatus generating a pulsed magnetic field, wherein the magnetic therapy apparatus includes: a coil ring for generating a magnetic field; and a magnetic field generating circuit passing a current through the coil ring to generate the magnetic field, and the magnetic field generating circuit passes a pulsed current having a pulse width of 0.5 msec or more and 5.0 msec or less through the coil ring to generate the magnetic field having a magnetic flux density of 50 mT or more and 300 mT or less.

A second aspect is the magnetic therapy apparatus according to the first aspect, wherein a frequency of the current passed by the magnetic field generating circuit through the coil ring is 1 Hz or more and 30 Hz or less.

A third aspect is the magnetic therapy apparatus according to the first aspect, wherein a frequency of the current passed by the magnetic field generating circuit through the coil ring varies in a predetermined frequency band within a range of 1 Hz or more and 10 Hz or less.

A fourth aspect is the magnetic therapy apparatus according to the third aspect, wherein the frequency of the current passed by the magnetic field generating circuit through the coil ring varies in a frequency band of 3 Hz or more and 5 Hz or less.

A fifth aspect is the magnetic therapy apparatus according to the fourth aspect, wherein the frequency of the current passed by the magnetic field generating circuit through the coil ring varies in a pattern of 1/f fluctuation.

A sixth aspect is the magnetic therapy apparatus according to any one of the first to fifth aspects, wherein the magnetic field generating circuit includes a capacitor and a thyristor connected in series with the coil ring.

Effects of the Invention

According to the magnetic therapy apparatus according to the first to sixth aspects, the pulsed current having the pulse width of 0.5 msec or more and 5.0 msec or less is passed through the coil ring to generate the magnetic field having the magnetic flux density of 50 mT or more and 300 mT or less, and thus, by providing the magnetic field to body tissue, an electromotive force having a similar waveform to an action potential is generated in the body tissue, and a magnetic therapy effect can be obtained while presenting less discomfort.

In particular, according to the magnetic therapy apparatus according to the fifth aspect, the frequency of the current passed through the coil ring varies in the pattern of the 1/f fluctuation, and thus discomfort presented by the magnetic field can be reduced.

Brief Description of Drawings

FIG. 1 is a perspective view illustrating an external appearance of a magnetic therapy apparatus according to the present invention as a whole.
FIG. 2 illustrates a configuration of a ring.
FIG. 3 shows a magnetic field generating circuit.
FIG. 4 shows a waveform of a pulsed current flowing through a coil ring.
FIG. 5 shows a change of an induced electromotive force induced by a change of a magnetic flux generated from the coil ring.

Description of Embodiments

An embodiment of the present invention will be described in detail below with reference to the drawings.

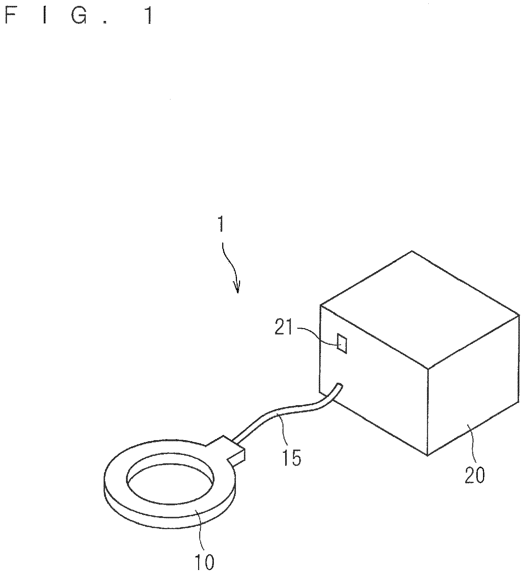

FIG. 1 is a perspective view illustrating an external appearance of a magnetic therapy apparatus 1 according to the present invention as a whole. The magnetic therapy apparatus 1 has a configuration in which a ring 10 and a body 20 are connected by a cable 15. In FIG. 1 and the subsequent drawings, the dimensions and the number of parts are exaggerated or simplified as necessary for ease of understanding.

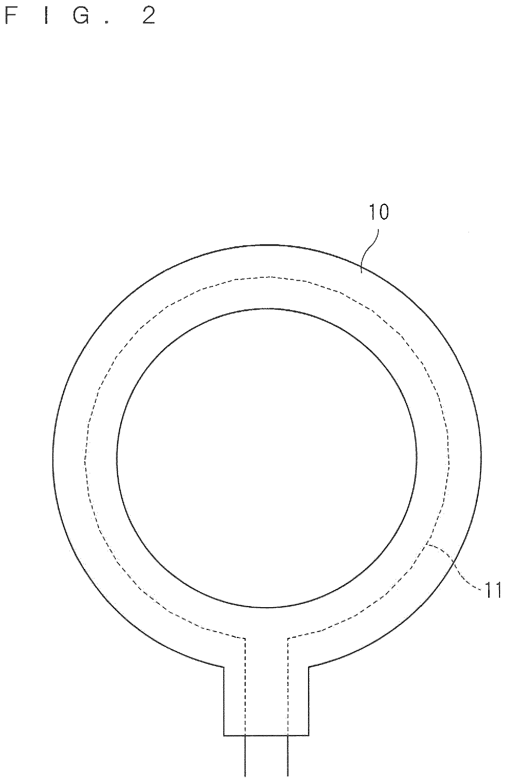

FIG. 2 illustrates a configuration of the ring 10. The ring 10 has a configuration in which a coil ring 11 is incorporated in an annular case made of resin, for example. The coil ring 11 is a coil in which the number of turns is one. The coil ring 11 is connected to the body 20 by the cable 15. The number of turns in the coil ring 11 may be two or more.

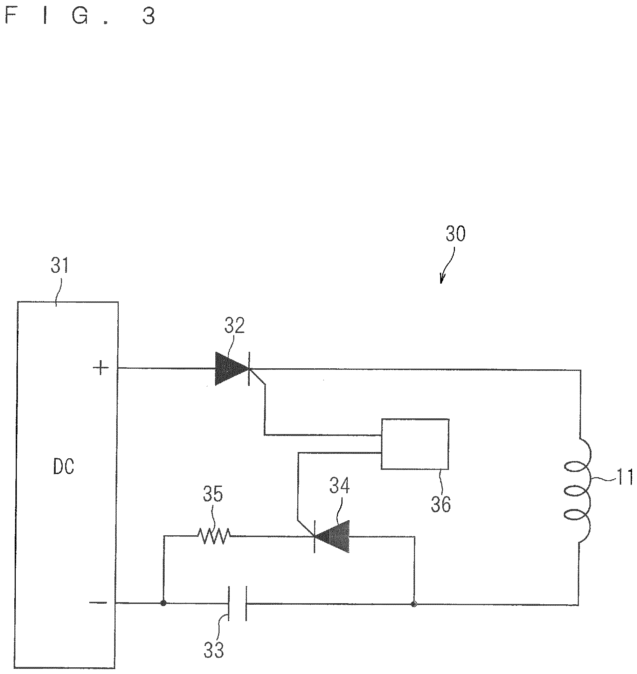

The body 20 incorporates therein a magnetic field generating circuit 30 for passing a pulsed current through the coil ring 11 to generate a pulsed magnetic field. FIG. 3 shows the magnetic field generating circuit 30. A portion of a circuit diagram shown in FIG. 3 excluding the coil ring 11 is incorporated in the body 20.

The magnetic field generating circuit 30 provided in the body 20 includes a DC power supply 31, a first thyristor 32, a capacitor 33, a second thyristor 34, a resistor 35, and a gate control device 36. The DC power supply 31 is a device supplying direct current electricity. The DC power supply 31 typically includes a transformer connected to an AC power supply (e.g., a household power supply) for stepping up or down, a rectifying circuit rectifying an alternating current, and a smoothing circuit smoothing the rectified current.

An anode of the first thyristor 32 is connected to a positive electrode of the DC power supply 31, and a cathode of the first thyristor 32 is connected to a positive electrode-side end of the coil ring 11. A negative electrode-side end of the coil ring 11 is connected to a positive electrode-side end of the capacitor 33, and a negative electrode-side end of the capacitor 33 is connected to a negative electrode of the DC power supply 31. That is to say, the first thyristor 32, the coil ring 11, and the capacitor 33 are connected in series.

The second thyristor 34 and the resistor 35 are connected in parallel with the capacitor 33. An anode of the second thyristor 34 is connected to the positive electrode-side end of the capacitor 33, and a cathode of the second thyristor 34 is connected to the negative electrode of the DC power supply 31 via the resistor 35.

The gate control device 36 is connected to gates of the first thyristor 32 and the second thyristor 34. The gate control device 36 passes gate currents (trigger currents) to the gates of the first thyristor 32 and the second thyristor 34 at a preset timing to control conduction of the first thyristor 32 and the second thyristor 34. The gate control device 36 supplies the gate currents individually to the first thyristor 32 and the second thyristor 34. As the gate control device 36, a one-chip microcomputer and the like can be used, for example.

When a switch 21 (see FIG. 1) of the body 20 is turned on, the DC power supply 31 applies a predetermined voltage to the magnetic field generating circuit 30. Unless a current is passed to the gate of the first thyristor 32, the first thyristor 32 is always off and not in a conducting state even if a voltage is applied in a forward or backward direction. Conduction of the first thyristor 32 starts when the gate control device 36 passes a current into the gate of the first thyristor 32 while the DC power supply 31 is in an on state and the first thyristor 32 is at a higher pressure on an anode side than on a cathode side. When the first thyristor 32 is in the conducting state, a current flows through the first thyristor 32 in the forward direction, that is, from the anode side to the cathode side.

When conduction of the first thyristor 32 starts, a current flows through the coil ring 11 from the DC power supply 31 via the first thyristor 32. When the current flows through the coil ring 11, a magnetic field is generated around the ring 10. The current flowing through the coil ring 11 flows into the capacitor 33, and is stored as a charge in the capacitor 33. As the charge is stored in the capacitor 33, an electromotive force is generated between electrodes of the capacitor 33, and the flow of the current stops when a voltage generated by the charge stored in the capacitor 33 becomes equal to a voltage applied by the DC power supply 31. When the flow of the current stops, the first thyristor 32 is turned off, and is not in the conducting state unless a signal is applied to the gate again. The current thus does not flow through the coil ring 11.

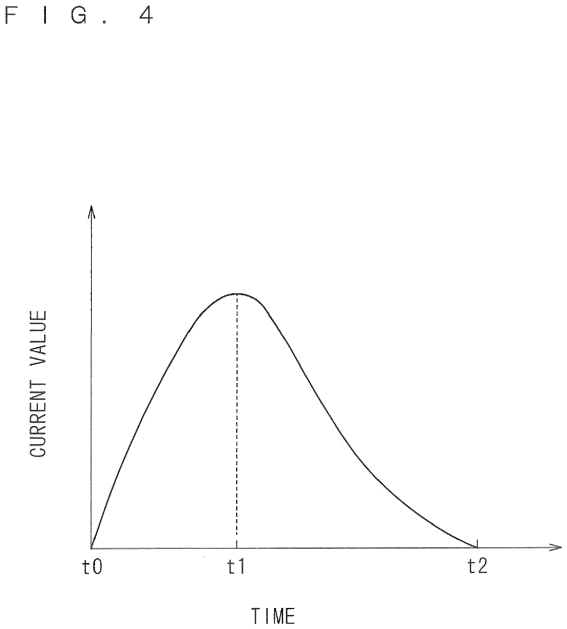

The pulsed current flows through the coil ring 11 once as described above. FIG. 4 shows a waveform of the pulsed current flowing through the coil ring 11. When the gate control device 36 passes the current into the gate of the first thyristor 32 at a time t0 while the DC power supply 31 is in the on state, conduction of the first thyristor 32 starts, and the current starts to flow through the coil ring 11. When the current flows through the coil ring 11, a magnetic flux is generated. A magnetic flux Φ (Wb) inside the coil ring 11 generated when the current flows through the coil ring 11 is expressed by an equation (1) shown below. In the equation (1), L is self-inductance of the coil ring 11, and I is a current value (i.e., the vertical axis in FIG. 4) of the current flowing through the coil ring 11.

[Math 1]

$$\Phi = L \times I \qquad \cdots (1)$$

As shown in the equation (1), the magnetic flux Φ generated when the current flows through the coil ring 11 is proportional to the current I flowing through the coil ring 11. As shown in FIG. 4, the flowing current I increases with increasing time elapsed from the start of the flow of the current through the coil ring 11 at the time t0. As the current I flowing through the coil ring 11 increases, the magnetic flux Φ increases in proportion to the current I, and, by electromagnetic induction caused by the change of the magnetic flux Φ, an induced electromotive force is generated in a direction to block the change of the current flowing through the coil ring 11 (so-called self-induction). The current flowing through the coil ring 11 thus increases gradually to some extent immediately after the time t0.

The charge stored in the capacitor 33 increases as the time elapses from the time t0. The charge stored in the capacitor 33 generates an electromotive force in a direction to block the current flowing through the coil ring 11. Thus, as the charge stored in the capacitor 33 increases, a potential difference across the coil ring 11 decreases, and the current flowing through the coil ring 11 starts to decrease after reaching a maximum value at a time t1. At a time t2 when the electromotive force generated by the charge stored in the capacitor 33 becomes equal to the voltage applied by the DC power supply 31, the potential difference across the coil ring 11 becomes zero, and the flow of the current stops. That is to say, the current value of the current flowing through the coil ring 11 becomes zero. The first thyristor 32 is turned off when the flow of the current stops.

A waveform of the pulsed current as shown in FIG. 4 is formed through such a process. In the present invention, a pulse width of the pulsed current flowing through the coil ring 11 is set to a time period from the time t0 when the current starts to flow through the coil ring 11 to the time t2 when the flow of the current stops. The above-mentioned phenomenon after the start of conduction of the first thyristor 32 is completed in an extremely short time, and the pulse width of the pulsed current flowing through the coil ring 11 is 0.5 msec or more and 5.0 msec or less in the present embodiment.

The magnetic field generated by the above-mentioned pulsed current flowing through the coil ring 11 has a magnetic flux density of 50 mT (500 G) or more and 300 mT (3000 G) of less. That is to say, the magnetic field generating circuit 30 passes the pulsed current having a pulse width of 0.5 msec or more and 5.0 msec or less through the coil ring 11 to generate the magnetic field having a magnetic flux density of 50 mT or more and 300 mT of less.

As shown in the equation (1), the magnetic flux Φ generated from the coil ring 11 is proportional to the current I flowing through the coil ring 11. Thus, when the current I flowing through the coil ring 11 changes as shown in FIG. 4, the magnetic flux Φ generated from the coil ring 11 changes over time to have a similar waveform.

When the magnetic flux Φ generated from the coil ring 11 changes over time in a case where a measurement coil is provided to face the coil ring 11, an induced electromotive force V generated in the measurement coil is expressed by an equation (2) shown below. The equation (2) is an equation representing Faraday's law of electromagnetic induction. In the equation (2), N is the number of turns in the measurement coil, and t is a time.

[Math 2]

$$V = -N \frac{d\Phi}{dt} \qquad \cdots (2)$$

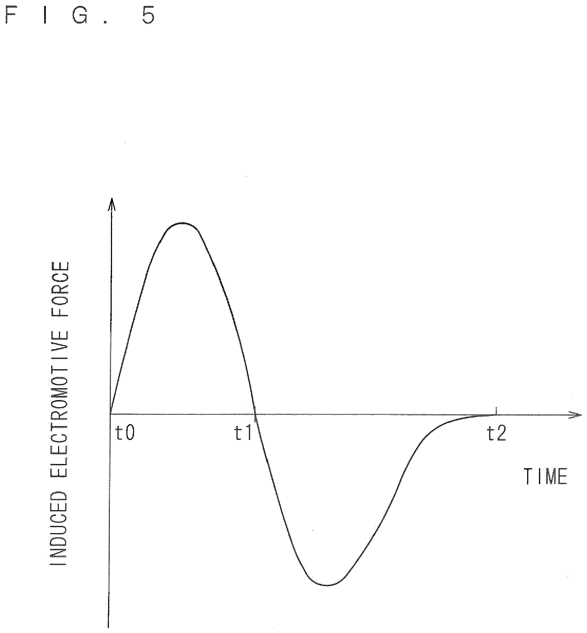

FIG. 5 shows a change of the induced electromotive force V induced by the change of the magnetic flux Φ generated from the coil ring 11. As shown in the equation (2), the induced electromotive force V is a time derivative of the change of the magnetic flux generated from the coil ring 11, and thus has opposite signs when the magnetic flux $\Phi$ increases and when the magnetic flux $\Phi$ decreases as the time elapses. The magnetic flux $\Phi$ is proportional to the current I flowing through the coil ring 11, and thus the induced electromotive force V has opposite signs when the current I flowing through the coil ring 11 increases and when the current I flowing through the coil ring 11 decreases. Thus, as shown in FIG. 5, the induced electromotive force V generated in the measurement coil has opposite signs in a time period from the time t0 to the time t1 during which the current I increases and in a time period from the time t1 to the time t2 during which the current I decreases when the above-mentioned pulsed current flows through the coil ring 11.

While FIG. 5 shows the change of the induced electromotive force V generated in the measurement coil provided to face the coil ring 11, an electromotive force having a similar waveform to that in FIG. 5 is induced in body tissue, and a weak current flows when the coil ring 11 generating the magnetic field is moved closer to the human body.

The waveform in FIG. 5 is herein similar to a waveform of a change of an action potential. The action potential refers to a membrane potential generated when cells and tissue of a living organism receive any stimulation. A portion exited upon receipt of stimulation has a negative potential with respect to the other portion to generate the action potential. As with the waveform shown in FIG. 5, the action potential rises significantly, undershoots, and then returns to zero. A time period from the rise to the return to zero of the action potential is similar to a time period from the rise to the return to zero of the induced electromotive force V shown in FIG. 5, that is, the pulse width of the pulsed current flowing through the coil ring 11 (the time period from the time t0 to the time t2). That is to say, the waveform of the electromotive force induced in the body tissue by the coil ring 11 generating the magnetic field and a time period during which the electromotive force is generated are respectively similar to the waveform of the action potential and the duration of the action potential.

Although the exact mechanism has not been elucidated, when an electromotive force having a waveform and duration similar to those of an action potential is induced in body tissue, the electromotive force is less likely to provide a stimulus to the body tissue. Thus, by providing the pulsed magnetic field from the coil ring 11 of the magnetic therapy apparatus 1 according to the present invention to the body tissue, a magnetic therapy effect can be obtained while presenting less discomfort.

In the present embodiment, a frequency at which the pulsed magnetic field is generated, that is, a frequency of the pulsed current passed by the magnetic field generating circuit 30 through the coil ring 11 varies in a frequency band of 3 Hz or more and 5 Hz or less. That is to say, the frequency of the pulsed current passed through the coil ring 11 varies over time within a range of three to five times per second. The pulsed current starts to flow when conduction of the first thyristor 32 starts upon application of an electric signal to the gate of the first thyristor 32, and thus a frequency at which the gate control device 36 passes the current into the gate of the first thyristor 32 as it is becomes the frequency of the pulsed current flowing through the coil ring 11. That is to say, the frequency of the pulsed current passed through the coil ring 11 can be controlled by the gate control device 36.

When the pulsed current flows through the coil ring 11 once, the charge is stored and remains in the capacitor 33, and thus a new pulsed current does not flow even if the current is passed into the gate of the first thyristor 32 in this state to cause the first thyristor 32 to be in the conducting state. A residual charge stored in the capacitor 33 is thus discharged immediately after the time t2 when the current value of the pulsed current becomes zero. Specifically, the gate control device 36 passes the current into the gate of the second thyristor 34 immediately after the time t2 to cause the second thyristor 34 to be in the conducting state. The residual charge stored in the capacitor 33 is thereby consumed by the resistor 35, and discharged. In other words, the second thyristor 34 and the resistor 35 constitute a discharge circuit for discharging the residual charge in the capacitor 33. The gate control device 36 passes the current into the gate of the first thyristor 32 after the residual charge stored in the capacitor 33 is discharged to allow a new pulsed current to flow through the coil ring 11.

In the present embodiment, the frequency of the pulsed current flowing through the coil ring 11 varies in the frequency band of 3 Hz or more and 5 Hz or less as described above, and a pattern of the variation is set to a pattern of 1/f fluctuation. The 1/f fluctuation is fluctuation inversely proportional to the frequency. That is to say, when the frequency at which the gate control device 36 passes the current into the gate of the first thyristor 32 is varied in the frequency band of 3 Hz or more and 5 Hz or less in the pattern of the 1/f fluctuation, the frequency of the pulsed current flowing through the coil ring 11 synchronizes with the frequency.

By varying the frequency of the pulsed current flowing through the coil ring 11 in the frequency band of 3 Hz or more and 5 Hz or less in the pattern of the 1/f fluctuation, discomfort presented by the pulsed magnetic field can be reduced.

While the embodiment of the present invention has been described so far, various modifications other than those described above can be made without departing from the scope of the present invention. For example, although the frequency of the pulsed current passed through the coil ring 11 is varied in the frequency band of 3 Hz or more and 5 Hz or less in the pattern of the 1/f fluctuation in the above-mentioned embodiment, the frequency may not be varied in this manner, and may be simply linearly varied in the frequency band.

Furthermore, in a case where the frequency of the pulsed current passed through the coil ring 11 is varied, the frequency band of the variation is not limited to 3 Hz or more and 5 Hz or less, and the frequency is only required to be varied in a predetermined frequency band within a range of 1 Hz or more and 10 Hz or less. For example, the frequency of the pulsed current passed through the coil ring 11 may vary in a frequency band of 2 Hz or more and 6 Hz or less or in a frequency band of 5 Hz or more and 9 Hz or less.

The frequency of the pulsed current passed through the coil ring 11 may not vary, and may be fixed. The frequency of the pulsed current passed through the coil ring 11 is only required to be 1 Hz or more and 30 Hz or less in a case where it is fixed.

The magnetic field generating circuit 30 in the above-mentioned embodiment is a combination of the thyristors and the capacitor, but is not limited to the magnetic field generating circuit having this configuration, and may have any circuit configuration in which the pulsed current can be passed through the coil ring 11 at a relatively low frequency of 30 Hz or less.

Explanation of Reference Signs

    1 magnetic therapy apparatus
    10 ring
    11 coil ring
    20 body
    30 magnetic field generating circuit
    31 DC power supply
    32 first thyristor
    33 capacitor
    34 second thyristor
    35 resistor
    36 gate control device

**Claims**

1.   A magnetic therapy apparatus generating a pulsed magnetic field, the magnetic therapy apparatus comprising:

       a coil ring for generating a magnetic field; and
       a magnetic field generating circuit passing a current through said coil ring to generate the magnetic field, wherein said magnetic field generating circuit passes a pulsed current having a pulse width of 0.5 msec or more and 5.0 msec or less through said coil ring to generate the magnetic field having a magnetic flux density of 50 mT or more and 300 mT or less.

2.   The magnetic therapy apparatus according to claim 1, wherein
     a frequency of the current passed by said magnetic field generating circuit through said coil ring is 1 Hz or more and 30 Hz or less.

3.   The magnetic therapy apparatus according to claim 1, wherein
     a frequency of the current passed by said magnetic field generating circuit through said coil ring varies in a predetermined frequency band within a range of 1 Hz or more and 10 Hz or less.

4.   The magnetic therapy apparatus according to claim 3, wherein
     the frequency of the current passed by said magnetic field generating circuit through said coil ring varies in a frequency band of 3 Hz or more and 5 Hz or less.

5.   The magnetic therapy apparatus according to claim 4, wherein
     the frequency of the current passed by said magnetic field generating circuit through said coil ring varies in a pattern of 1/f fluctuation.

6.   The magnetic therapy apparatus according to any one of claims 1 to 5, wherein
     said magnetic field generating circuit includes a capacitor and a thyristor connected in series with said coil ring.

F I G . 1

1

21

15

10

20

F I G . 2

F I G . 3

F I G . 4

F I G . 5

## INTERNATIONAL SEARCH REPORT

International application No.

PCT/JP2017/016169

| A. CLASSIFICATION OF SUBJECT MATTER |
|---|
| A61N2/04(2006.01)i |

According to International Patent Classification (IPC) or to both national classification and IPC

| B. FIELDS SEARCHED |
|---|
| Minimum documentation searched (classification system followed by classification symbols) |
| A61N2/04 |

Documentation searched other than minimum documentation to the extent that such documents are included in the fields searched

Jitsuyo Shinan Koho 1922–1996 Jitsuyo Shinan Toroku Koho 1996–2017
Kokai Jitsuyo Shinan Koho 1971–2017 Toroku Jitsuyo Shinan Koho 1994–2017

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | "Saiboryoku Kenkoho Saiboryoku Iji de Kenko ni!! - Cell Power", [online], 02 July 2015 (02.07.2015), [retrieval date 24 November 2016 (24.11.2016)], Internet: <URL:https://web.archive.org/web/20150702123718/http://www.cellpower.jp/about> | 1-6 |
| Y | "Hito o Kenko ni suru Hasso ga Unda Shinkeiha Jiryokusen Hasseiki", [online], 07 January 2015 (07.01.2015), [retrieval date 24 November 2016 (24.11.2016)], Internet: <URL:http://ameblo.jp/koji-kitano/entry-11970148523.html> | 1-6 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 09 June 2017 (09.06.17) | 27 June 2017 (27.06.17) |

| Name and mailing address of the ISA/ | Authorized officer |
|---|---|
| Japan Patent Office 3-4-3,Kasumigaseki,Chiyoda-ku, Tokyo 100-8915,Japan | |
| | Telephone No. |

Form PCT/ISA/210 (second sheet) (January 2015)

**INTERNATIONAL SEARCH REPORT**

International application No.

PCT/JP2017/016169

C (Continuation).    DOCUMENTS CONSIDERED TO BE RELEVANT

| Category* | Citation of document, with indication, where appropriate, of the relevant passages | Relevant to claim No. |
|---|---|---|
| Y | JP 3053000 U  (Ikari Kankyo Service Kabushiki Kaisha),<br>13 October 1998 (13.10.1998),<br>paragraph [0002]<br>(Family: none) | 3-5 |
| Y | JP 8-152929 A  (Takeshi HAYASHIBARA),<br>11 June 1996 (11.06.1996),<br>paragraph [0002]; fig. 3<br>& US 5691873 A<br>column 1, lines 13 to 31; fig. 3<br>& DE 69501141 T2          & EP 0704865 A1 | 6 |
| A | US 5030196 A  (INOUE-JAPAX RESEARCH INC.),<br>09 July 1991 (09.07.1991),<br>column 7, line 48 to column 9, line 3; fig. 9 to 10<br>& EP 0039206 A1 | 1-2 |
| A | US 5984854 A  (NIHON KOHDEN CORP.),<br>16 November 1999 (16.11.1999),<br>column 4, line 61 to column 6, line 26; fig. 1 to 4<br>& EP 0788813 A1          & EP 1319422 A2 | 1-2 |
| A | JP 2008-280564 A  (Koji SASAKI),<br>20 November 2008 (20.11.2008),<br>paragraphs [0034] to [0048]; fig. 3<br>(Family: none) | 6 |
| E,X | JP 6138306 B1  (CellPower Co., Ltd.),<br>31 May 2017 (31.05.2017),<br>entire text; all drawings<br>(Family: none) | 1-6 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015077484 A
- JP 2012511387 W